Europäisches Patentamt

**European Patent Office**

**Office européen des brevets**

(11) Numéro de publication: **0 127 557**

**A1**

(12) **DEMANDE DE BREVET EUROPEEN**

(21) Numéro de dépôt: **84430012.9**

(22) Date de dépôt: **25.04.84**

(51) Int. Cl.³: **A 61 L 9/01**
**C 11 D 3/50, A 01 N 59/00**

(30) Priorité· **25.04.83 FR 8306886**

(43) Date de publication de la demande:
**05.12.84 Bulletin 84/49**

(84) Etats contractants désignés:
**BE CH DE GB IT LI NL SE**

(71) Demandeur: **Pecqueux, Thierry**
**70 Val Saint Georges**
**F-13170 La Gavotte(FR)**

(72) Inventeur: **Pecqueux, Thierry**
**70 Val Saint Georges**
**F-13170 La Gavotte(FR)**

(74) Mandataire: **Roman, Alphonse**
**35 Rue Paradis**
**F-13001 Marseille(FR)**

(54) **Produit pour le traitement des surfaces absorbantes ayant reçu des égouttures de graisse animale brute.**

(57) Produit renferment du peroxyde d'hydrogène et du dioc-tylsulfosuccinate de sodium mélangé à de l'eau douce avec un parfum d'appoint pour le traitement permettant de transformer la graisse animale ayant pénétré dans une surface absorbante afin de la débarrasser de leur odeur et plus particulièrement ceux des conteneurs.

EP 0 127 557 A1

- 1 -

<u>Produit pour le traitement des surfaces absorbantes
ayant reçu dees égouttures de graisses animale brute</u>

Produit pour le traitement des surfaces absorbantes
ayant reçu des égouttures de graisse animale brute.

Il est destiné au traitement permettant de transformer la graisse animale ayant pénétré dans la fibre de bois des planchers afin de la débarrasser de leur odeur et plus particulière ment ceux des conteneurs.

Actuellement on ne procède pas d'une façon efficace au nettoyage, on a la désodorisation des planchers en bois souillés par des graisses animales brutes. Il faudrait en effet extraire ou dissoudre la graisse ayant pénétré au coeur du bois, ce qui peut se faire sans attaquer ou désagréger le support. On a alors essayé de masquer l'odeur désagréable émise, par un parfumage intense. Mais à ce jour, pour arriver à un résultat positif, on change le plancher pollué.

Le produit suivant l'invention supprime ces inconvénients et permet de faire disparaitre l'odeur des égouttures de graisse animale brute qui émane du plancher souillé par un raffinage supprimant leur odeur au moyen du blanchiment et de la destruction des bactéries graisseuses qui imbibent le bois du plancher.

Il est constitué par l'application nouvelle du peroxyde d'hydrogène, du dioctylsulfosuccinate de sodium mélangé à de l'eau douce avec un parfum d'appoint.

Suivant un mode de réalisation du produit donné à titre d'exemple non limitatif.

Les agents sont utilisés dans les proportions suivantes pour obtenir une solution fluide :

1° Peroxyde d'hydrogène 30 % en poids
2° Dioctylsulfosuccinate de sodium 1 % en poids
3° Parfum synthétique 0,5 % en poids
4° Eau douce 68,5 % en poids

Ces agents sont mélangés à froid dans un malaxeur jusqu'à l'obtention d'une répartition homogène dans l'eau douce.

Le peroxyde d'hydrogène joue le rôle d'oxydant fort et assure la destruction des bactéries et des matières en décomposition de la graisse animale brute.

Le dioctylsulfosuccinate de soude abaisse la tension superficielle du mélange provoquant la pénétration rapide du substrat ligneux.

Le parfum permet d'identifier les planchers recemment traités.

La solution se présente sous forme de liquide incolore, de viscosité proche de celle de l'eau. on peut ainsi utiliser pour son répandage, aussi bien un arrosoir qu'un pulvérisateur ou atomiseur.

Il est utilisable pour le traitement des conteneurs ayant transporté des peaux de mouton, pour les plateaux de camions, de wagons et les cales de navires.

Son action est efficace pour les locaux souillés par des graisses animales brutes et toutes les fois qu'on veut entreposer ou transporter des marchandises autres, lorsque l'odeur peut endommager un produit susceptible d'être détérioré par le contact avec les surfaces portantes imprégnées.

Les quantités et natures des agents pourront varier dans la limite des équivalents comme d'ailleurs les matières secondaires utilisées, sans changer pour cela, la conception générale de l'invention qui vient d'être décrite.

- 1 -

Revendications de brevet

1. Produit pour le traitement des surfaces absorbantes ayant reçu des égouttures de graisse animale brute permettant par raffinage sur la place réceptrice de son répandage la suppression des odeurs, se caractérisant par une solution à base de peroxyde d'hydrogène, de dioctylsulfosuccinate de sodium, d'eau et de parfum signalisateur de traitement.

2. Produit suivant la revendication 1 se caractérisant par le mélange et le mixage à froid dans 68,5 % d'eau douce en poids, avec 30 % en poids de peroxyde d'hydrogène, 1 % en poids de dioctylsulfosuccinate de sodium, le tout complété par 0,5 % en poids de parfum.

Office européen
des brevets

**RAPPORT DE RECHERCHE EUROPEENNE**

## DOCUMENTS CONSIDERES COMME PERTINENTS

| Catégorie | Citation du document avec indication en cas de besoin, des parties pertinentes | Revendication concernee | CLASSEMENT DE LA DEMANDE (Int Cl 4) |
|---|---|---|---|
| A | GB-A-1 562 419 (FUMAKILLA LTD.) * En entier * | 1,2 | A 61 L 9/01<br>C 11 D 3/50<br>A 01 N 59/00 |
| A | GB-A- 886 084 (DOMESTOS LTD.) * Revendications * | 1,2 | |
| A | CHEMICAL ABSTRACTS, vol. 90, no. 6, 5 février 1979, pages 64-65, no. 40162u, Columbus, Ohio, US; & PL - A - 88 197 (ZAKLADY PRZEMYSLU BAWELNIANEGO IM. STANISLAWA DUBOIS) 30-12-1976 * En entier * | 1,2 | |

| DOMAINES TECHNIQUES RECHERCHES (Int. Cl. 3) |
|---|
| A 61 L<br>C 11 D<br>A 01 N |

Le present rapport de recherche a ete etabli pour toutes les revendications

| Lieu de la recherche | Date d'achèvement de la recherche | Examinateur |
|---|---|---|
| LA HAYE | 31-07-1984 | FLETCHER A.S. |